# EUROPÄISCHE PATENTANMELDUNG

(11) **EP 4 641 185 A1**
(43) Veröffentlichungstag der Anmeldung: **29.10.2025**
(21) Anmeldenummer: 24172122.4
(22) Anmeldetag: 24.04.2024
(51) Int. Cl.: G01N 23/04

(54) **VERFAHREN UND VORRICHTUNG ZUR UNTERSUCHUNG VON PRODUKTEN**

(71) Anmelder: Sesotec GmbH, 94513 Schönberg (DE)
(72) Erfinder: Kurz, Reinhard, 94269 Rinchnach (DE); Eder, Johann, 94513 Schönberg (DE)
(74) Vertreter: Glück Kritzenberger Patentanwälte PartGmbB

(57) **Zusammenfassung**

Vorrichtung (10) zur Erfassung von Fremdstoffen in Produkten (13), insbesondere Lebensmitteln, umfassend eine Röntgen-Detektionsvorrichtung (12) mit einer Röntgenquelle (15) und einem Röntgendetektor (16), und ein Fördermittel (14) zum Fördern der Produkte durch einen Erfassungsbereich (30) des Röntgendetektors (16). Erfindungsgemäß werden produktfreie Bereiche (a) des Fördermittels (14) von der Röntgen-Detektionsvorrichtung (12) im laufenden Betrieb detektiert und die erhaltenen Signalwerte werden pixelbasiert mit einem kalibrierten Sollwert verglichen werden, wobei ein Warnsignal abgegeben wird, wenn die Abweichung des Signalwerts zumindest eines Pixels (34) von dem kalibrierten Sollwert einen Grenzwert überschreitet.

## Beschreibung

Die vorliegende Erfindung betrifft ein Verfahren und eine Vorrichtung zur Erfassung von Fremdstoffen in Produkten, insbesondere Lebensmitteln, umfassend eine Röntgen-Detektionsvorrichtung mit einer Röntgenquelle und einem Röntgendetektor, und ein Fördermittel zum Fördern der Produkte durch einen Erfassungsbereich des Röntgendetektors. Die Röntgen-Detektionsvorrichtung ist ausgebildet, das Fördermittel selbst ohne aufliegende Produkte zu detektieren, um ein pixelbasiertes Detektionsbild entsprechend der Auflösung des Röntgendetektors zu erhalten. Die Vorrichtung weist eine Auswerteinheit auf, die ausgebildet ist, beim Kalibrieren der Vorrichtung die Signalwerte jedes Pixels des Röntgendetektors auf einen Sollwert zu normieren.

Eine derartige Vorrichtung ist sehr gut in der Lage, Fremdstoffe, insbesondere metallhaltige Fremdstoffe, in Produkten, vorzugsweise Lebensmitteln, zu erfassen, da derartige Fremdstoffe insbesondere bei Lebensmitteln durchaus bei der Bearbeitung, zum Beispiel beim Schneiden oder Verpacken, in das Lebensmittel gelangen können.

Ein Problem bei einer derartigen Vorrichtung besteht darin, dass im Betrieb das Fördermittel, welches in der Regel als Förderband ausgebildet ist, verschmutzen kann, wodurch die Detektionsgenauigkeit vermindert werden kann.

Es ist daher Aufgabe der vorliegenden Erfindung, eine Vorrichtung und ein Verfahren der oben genannten Art zu schaffen, die in der Lage sind, die Beibehaltung einer hohen Detektionsgenauigkeit der Vorrichtung sicherzustellen.

Diese Aufgabe wird durch eine Vorrichtung gemäß Anspruch 1 und durch ein Verfahren gemäß Anspruch 9 gelöst. Vorteilhafte Weiterbildungen der Erfindung sind Gegenstand der zugeordneten abhängigen Ansprüche. Vorteilhafte Weiterbildungen und Ausführungen der Erfindung sind auch Gegenstand der Beschreibung und der Zeichnung.

Erfindungsgemäß ist die Vorrichtung ausgebildet, im laufenden Betrieb mittels der Röntgen-Detektionsvorrichtung produktfreie Bereiche des Fördermittels zu scannen und Abweichungen der Signalwerte der in diesen Bereichen detektierten Pixel von dem Sollwert zu erfassen. Diese Abweichungen werden dann mittels der Auswertevorrichtung ausgewertet. Diese ist ausgebildet, ein Warnsignal abzugeben, wenn die Abweichung des Signalwertes eines oder mehrerer Pixel in den produktfreien Bereichen des Fördermittels von dem Sollwert einen Grenzwert überschreitet.

Diese technische Lösung ermöglicht im laufenden Betrieb eine ständige Überwachung des Fördermittels, in der Regel des Förderbandes, und stellt sicher, dass eventuelle Verschmutzungen des Förderbandes unterhalb eines bestimmten Grenzwerts bleiben. Wenn stärkere Verschmutzungen des Fördermittels vorkommen sollten, zum Beispiel durch einen Metalspan auf dem Förderband, wird ein Warnsignal abgegeben, so dass das Bedienpersonal in der Lage ist, das Fördermittel von den Verschmutzungen zu reinigen und eine hohe Detektionsqualität aufrechtzuerhalten. Durch die Erfindung werden somit Fehlauslösungen und eine Verschlechterung der Detektionsgenauigkeit des Röntgendetektors vermieden.

Der Grenzwert kann sich zum einen auf die Höhe der Abweichung eines Pixels von dem Sollwert beziehen. Er kann sich allerdings auch auf die Anzahl der vom Sollwert abweichenden Pixel beziehen, wobei in Abhängigkeit von der Anzahl der abweichenden Pixel durchaus unterschiedliche Grenzwerte verwendet werden können, in dem Sinn, dass bei großflächigeren Abweichungen bereits ein geringerer Unterschied zwischen den Pixelwerten und dem Sollwert ausreichend ist, um ein Warnsignal abzugeben.

Prinzipiell kann das Warnsignal ein akustisches und/oder visuelles Signal sein, das das Bedienpersonal der Vorrichtung auffordert, das Fördermittel zu reinigen. Am besten wird dies durch ein Display angezeigt, das mit einer Steuerung der Vorrichtung verbunden ist.

Eine derartige Vorrichtung hat in der Regel auch eine Produktweiche, die durch die Auswerteeinheit angesteuert wird. Durch die laufende Überwachung des Fördermittels im Betrieb wird sichergestellt, dass das durch das Fördermittel verursachte Hintergrundsignal in engen Toleranzgrenzen bleibt, womit die Auswerteeinheit bessere Detektionsergebnisse liefert und somit die Zuordnung zwischen Gutprodukten und Schlechtprodukten mittels der Produktweiche erheblich verbessert wird.

Vorzugsweise ist der Röntgendetektor ausgebildet, das Fördermittel Zeile für Zeile zu erfassen oder abzutasten, wobei jede Zeile in Pixel unterteilt ist. So kann ein Detektionsbild beispielsweise entsprechend der Auflösung des Detektors 1000 Zeilen à 1000 Pixel enthalten oder ähnliche Werte.

In einer vorteilhaften Weiterbildung der Erfindung ist die Vorrichtung ausgebildet, die Signalwerte jedes Pixels beim Kalibrieren des Röntgengerätes mit einem Sollwert zu vergleichen und zur Erzielung des Sollwertes mit einem Verstärkungsfaktor zu verstärken, der die Differenz zwischen dem Signalwert und dem Sollwert widerspiegelt. Die Auswerteeinheit ist in diesem Fall ausgebildet, ein Warnsignal abzugeben, wenn der Signalwert des Pixels nach der Normierung beziehungsweise trotz der Normierung von dem Sollwert abweicht. Dies ist ein Indiz dafür, dass das Fördermittel an der entsprechenden Stelle so stark verschmutzt oder beschädigt ist, dass eine Normierung im Rahmen des Kalibrierens nicht durchgeführt werden kann. In diesem Fall kann also die Qualität des Fördermittels zum einen beim Kalibrieren und zum anderen im laufenden Betrieb sichergestellt werden.

Vorzugsweise enthält die Vorrichtung eine Positionserkennungseinrichtung für die Position des Pixels mit dem abweichenden Signalwert. Diese Positionserkennungseinrichtung kann im einfachsten Fall ein Softwaremodul sein, das erkennt, welcher Pixel in einer Zeile des Erfassungsbereichs des Röntgendetektors von der Sollwertabweichung betroffen ist. Auf diese Weise lässt sich die Fehlerstelle quer zur Laufrichtung des Fördermittels sehr genau definieren. Die Positionserkennungseinrichtung kann so zum Beispiel über das Display der Steuerung der Vorrichtung anzeigen, an welcher Position quer zur Förderrichtung sich die Verschmutzung am Fördermittel befindet. Die Positionserkennungseinrichtung kann insofern vorzugsweise integriert sein in die Steuerung der Vorrichtung, die für die Steuerung des Fördermittels, des Röntgendetektors und gegebenenfalls der Produktweiche zuständig ist.

Im Falle eine Positionserkennungseinrichtung vorgesehen ist, kann diese auch eine Erkennung der aktuellen Position des Fördermittels umfassen. Dies kann zum Beispiel über die Erfassung einer Positionsmarkierung am Fördermittel erzielen, die in Laufrichtung des Fördermittels aufgebracht ist oder aber durch eine Markierung an einer einzigen Stelle des Fördermittels in Verbindung mit der Betätigung des Fördermittels zum Beispiel durch ein Schrittmotor oder durch eine Bewegungserkennung des Fördermittels. Derartige Längspositionserkennungen an Fördermitteln sind per se bekannt. Durch das Vorsehen einer derartigen Einrichtung im Rahmen der Positionserkennung lässt sich eindeutig genau die Stelle am Fördermittel anzeigen, an der eine Abweichung eines Pixels vorliegt, die zur Abgabe des Warnsignals geführt hat.

Vorzugsweise hat die Vorrichtung neben einer zentralen Steuerung auch ein Display, so dass beispielsweise über das Display die absolute Position einer Verschmutzung auf dem Förderband angezeigt werden kann, worauf eine Bedienperson entweder manuell oder automatisch diese Stelle anfahren kann.

Die Erfindung betrifft ebenfalls ein Verfahren zur Detektion von Fremdstoffen in Produkten, insbesondere Lebensmitteln, die in oben beschriebener Weise mittels eines Fördermittels durch einen Erfassungsbereich eines Röntgendetektors gefördert werden. Erfindungsgemäß werden produktfreie Bereiche des Fördermittels von der Röntgen-Detektionsvorrichtung im laufenden Betrieb detektiert und die Signalwerte werden pixelbasiert mit einem kalibrierten Sollwert verglichen, wobei ein Warnsignal abgegeben wird, wenn die Abweichung des Signalwertes zumindest eines Pixels von dem kalibrierten Sollwert einen Grenzwert überschreitet.

Hinsichtlich der Eigenschaften und Vorteile der Merkmale des Verfahrens wird auf die Beschreibung der erfindungsgemäßen Vorrichtung verwiesen. Beim Kalibrieren werden alle Pixel des Detektionswertes auf einen Sollwert verstärkt. Wenn nachher im laufenden Betrieb die Abweichung der detektierten Signalwerte der Pixel des produktfreien Fördermittels von diesem Sollwert den Grenzwert überschreitet, wird das Warnsignal akustischer und/oder optischer Art abgegeben, so dass das Betriebspersonal reagieren und zum Beispiel das Fördermittel säubern kann.

Wie oben bereits ausgeführt, kann der Grenzwert abhängig sein von der Anzahl der vom Sollwert abweichenden Pixel, insbesondere wenn die abweichenden Pixel nebeneinander liegen. So kann zum Beispiel ein geringerer Grenzwert vorgesehen sein, wenn eine nebeneinanderliegende zusammenhängende größere Fläche an Pixeln von dem Sollwert abweicht.

Das Fördermittel wird durch den Röntgendetektor insbesondere zeilenweise erfasst, wobei diese Zeilen insbesondere quer zur Förderrichtung liegen. Jede Zeile ist wiederum in Pixel unterteilt, so dass durch die einzelne Zeile und durch die Auswertung hintereinanderliegender Zeilen ein Bild des Fördermittels beziehungsweise der Produkte erhalten wird.

Beim Kalibrieren der Röntgen-Detektionsvorrichtung werden die Signalwerte jedes Pixels des Detektionsbildes des Röntgendetektors mit einem Sollwert verglichen und zur Erzielung des Sollwertes mit einem Verstärkungsfaktor verstärkt, welcher die Differenz zwischen dem Signalwert des entsprechenden Pixels und dem Sollwert widerspiegelt. Erfindungsgemäß wird nun ein Warnsignal abgegeben, wenn trotz der Normierung die Abweichung des Signalwert des Pixels vom Sollwert immer noch einen Grenzwert überschreitet. Dies zeigt nämlich an, dass die dem Pixel entsprechende Stelle des Fördermittels derart verschmutzt oder beschädigt ist, dass eine Normierung dieser Stelle nicht möglich ist. Somit wird bereits beim Kalibrieren das Fördermittel auf seine Fehlerhaftigkeit untersucht und eine hohe Qualität der Detektion sichergestellt.

In einer äußerst vorteilhaften Weiterbildung der Erfindung wird die aktuelle Position jedes Pixels des im insbesondere zeilenförmigen Erfassungsbereich des Röntgendetektors und/oder die aktuelle Position des Fördermittels erfasst. So ist es zumindest möglich, die exakte Position eines Fehlbereichs des Fördermittels quer zur Förderrichtung des Detektionsstrahls des Röntgendetektors in der Abtastzeile zu erfassen. Es kann sogar die absolute Position eines Fehlers erkannt werden, wenn zudem die aktuelle Position des Fördermittels ermittelt wird, und damit die Stelle des Fördermittels erfasst wird, die sich gerade im Erfassungsbereich des Röntgendetektors befindet. In diesem Fall ist es möglich, die absolute Position eines fehlerhaften Bereichs am Fördermittel, der das Warnsignal ausgelöst hat, anzuzeigen. Dies ermöglicht dem Fachpersonal ein sehr viel schnelleres Auffinden einer Verschmutzung oder Fehlstelle am Fördermittel, so dass einem derartigen Problem schneller abgeholfen werden kann.

Es ist für den Fachmann offensichtlich, dass die Merkmale der oben beschriebenen Vorrichtung und des Verfahrens, welches vorzugsweise die beschriebene Vorrichtung verwendet, in beliebiger Weise miteinander kombiniert werden können.

Die Erfindung wird nun nachfolgend beispielsweise anhand der schematischen Zeichnung beschrieben. In dieser zeigen:
- Fig. 1: eine schematische Ansicht einer Untersuchungsvorrichtung für Lebensmittel mit einem Förderband, einem Röntgendetektor und einer Produktweiche,
- Fig. 2: eine Aufsicht auf das Fördermittel in Richtung II aus Fig. 1, welche Verschmutzungen auf dem Fördermittel zeigt, die ein Warnsignal auslösen können,
- Fig. 3: eine Aufsicht auf das Band im Bereich des zeilenförmigen Erfassungsbereichs des Röntgendetektors, und
- Fig. 4: ein Detail IV aus Fig. 3.

Die erfindungsgemäße Lebensmittelinspektionsvorrichtung 10 umfasst eine Röntgen-Detektionsvorrichtung 12 mit einem Gehäuse 11, in welchem eine Röntgenquelle 15 und ein Röntgendetektor 16 angeordnet ist. Die Röntgenquelle 15 emittiert Röntgenstrahlung 17, welche ein Förderband 14 und darauf transportierte Produkte 13, insbesondere Lebensmittel, durchstrahlt.

Diese Strahlung trifft auf den Röntgendetektor 16 auf, der vorzugsweise als Liniendetektor ausgebildet ist.

Das Förderband 14, welches das Gehäuse der Röntgen-Detektionsvorrichtung 12 durchläuft, wird von einem Motor 18 betrieben, welcher von einer zentralen Steuerung 20 der Vorrichtung 10 gesteuert wird. Die zentrale Steuerung 20 steuert auch die Röntgen-Detektionsvorrichtung 12 und eine Produktweiche 22 am Ende des Förderbandes 14 an, um Gutprodukte von Schlechtproduktion abzutrennen.

Die zentrale Steuerung 20 enthält eine Auswerteeinheit 24, die in der Lage ist, die pixelbasierten Detektionssignale des Röntgendetektors 16 auszuwerten. Die Vorrichtung 10 ist in der Lage, den produktfreien Bereich a zwischen den Produkten 13 abzuscannen und jedes Pixel des produktfreien Fördermittelbereichs a mit einem kalibrierten Sollwert zu vergleichen. Wenn die Abweichung des Signalwertes eines Pixels und/oder einer Anzahl von Pixeln in dem produktfreien Bereich a den kalibrierten Sollwert einen Grenzwert überschreitet, wird ein Warnsignal abgegeben, welches beispielsweise über ein Display 26 der Steuerung 20 oder gegebenenfalls auch über ein akustisches Signal abgegeben werden kann. Vorzugsweise hat die Vorrichtung 10 eine Positionserkennungseinrichtung 25, die in der Lage ist, die absolute Position des Förderbandes in dem Erfassungsbereich zu erkennen. Auf diese Weise ist eine Verschmutzung am Förderband exakt lokalisierbar.

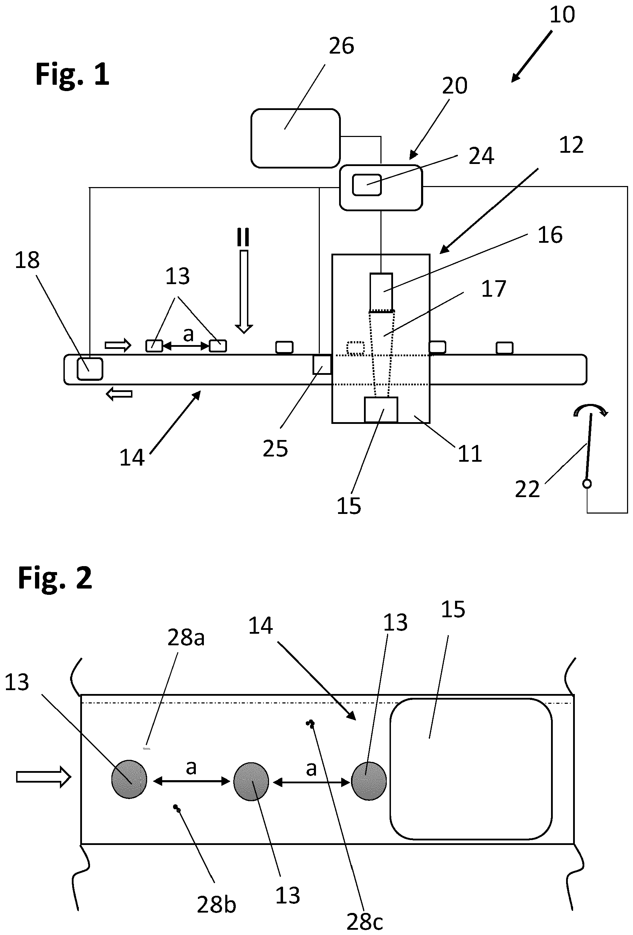

Figur 2 zeigt dahingehend eine Aufsicht II aus Figur 1 mit fehlerhaften Stellen 28a,b,c,d auf dem Förderband, die dazu geeignet sind, ein Warnsignal bei der Detektion auszulösen. Der Pfeil gibt die Laufrichtung des Bandes an. Die Röntgen-Detektionsvorrichtung 12 scannt im laufenden Betrieb ständig die produktfreien Bereiche a ab, und vergleicht die ermittelten Signalwerte der Pixel in diesen produktfreien Bereichen a mit dem kalibrierten Sollwert, um im Fall einer unzulässigen Abweichung eine Warnmeldung abgeben zu können. Auf diese Weise wird im laufenden Betrieb sichergestellt, dass eine gewisse Sauberkeit des Förderbandes 14 gewahrt bleibt, und dass Verschmutzungen, die das Detektionsergebnis beeinträchtigen könnten, sofort angezeigt werden.

Das Warnsignal wird somit ausgegeben, wenn wenigstens die Abweichung des Signalwertes eines Pixels von dem kalibrierten Sollwert einen Sollwert überschreitet. Wenn, wie das in Fig. 2 gezeigt ist, gleichzeitig drei Stellen 28a, 28b, 28c von dem Sollwert abweichen, oder eine Verschmutzung großflächiger ist, wie beispielsweise die Stelle 28c, kann der Grenzwert auch zur Größe der fehlerhaften Stellen korrelieren. So kann zum Beispiel bei einem großflächigeren Defekt oder bei einer größeren Anzahl an fehlerhaften Pixeln der Grenzwert herabgesetzt werden, so dass auch eine Gesamtverschmutzung mit an sich weniger stark beschädigten Einzelverschmutzungen erfasst und angezeigt werden kann.

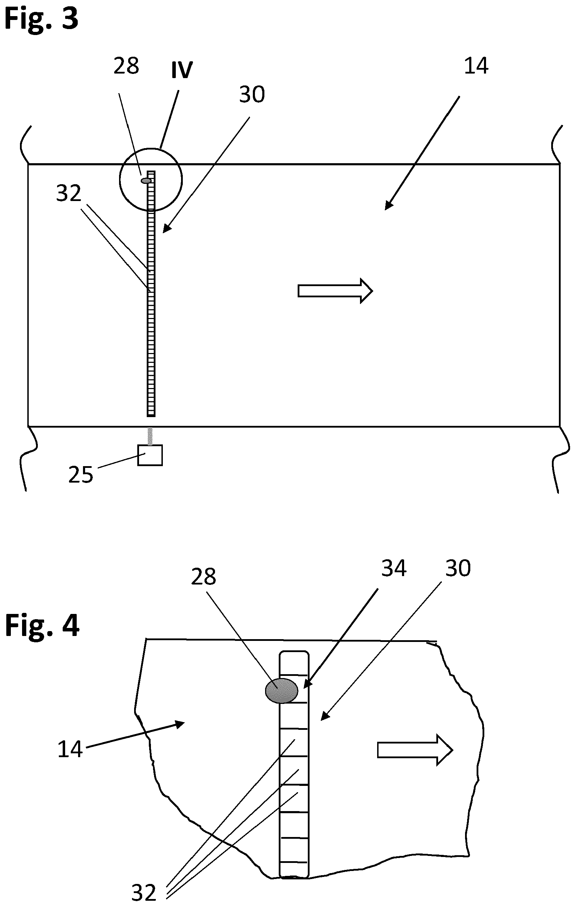

Figur 3 und 4 zeigen den zeilenförmigen Erfassungsbereich 30 des Röntgendetektors 16. Jede Abtastzeile 30 ist in eine Vielzahl an Pixeln 32 unterteil. Bei einem herkömmlichen Röntgendetektor können es mehrere 1000 sein. Das Detektionsbild wird erhalten, wenn in Laufrichtung des Förderbandes 14 hintereinander Zeile 30 für Zeile 30 durch den Erfassungsbereich 30 des Röntgendetektors 16 erfasst wird. Die Zeilen 30 sind dabei vorzugsweise direkt aneinandergrenzend, so dass als Detektionsbild eine flächige Pixelmatrix erhalten wird.

Wenn eine Verschmutzung 28 am Förderband 14 vorhanden ist, gelangt diese in den zeilenförmigen Abtastbereiche 30 und führt bei einem Pixel 34 zu einer stärkeren Abweichung des Signalwertes von dem kalibrierten Sollwert, so dass ein Warnsignal abgegeben wird, wenn die Abweichung des Signalwertes dieses Pixels 34 einen Grenzwert überschreitet. So kann eine Verschmutzung 28 des Förderbandes 14, insbesondere mit metallhaltigen Partikeln, welche die Detektionsgenauigkeit vermindern würden, sofort erkannt und beseitigt werden.

Durch die erfindungsgemäße Vorrichtung ist es damit möglich, im laufenden Betrieb den Zustand des Förderbandes 14 zu erkennen und somit sicherzustellen, dass dieses auf einem gewissen Sauberkeitsniveau verbleibt, um Fehlauslösungen bei der Produktzuweisung beziehungsweise bei der Fehlererkennung zu vermeiden, die letztendlich dazu führen können, dass irrtümlicherweise Gutprodukte durch entsprechende Betätigung der Produktweiche 22 als Schlechtprodukte abqualifiziert werden, wobei der Fehler dabei nicht am Produkt 13 sondern an einer Verschmutzung 28 des Förderbandes liegen würde. Somit wird durch die Erfindung die Detektionsleistung und die Detektionsqualität der Vorrichtung 10 beträchtlich verbessert.

Die Signalwerte werden regelmäßig als Helligkeitswert bzw. Grauwerte erfasst und dargestellt.

Die Erfindung ist nicht auf das dargestellte Ausführungsbeispiel begrenzt, sondern kann stattdessen innerhalb des Schutzbereichs der beiliegenden Ansprüche variiert werden.

### Bezugszeichenliste:

- 10: Untersuchungsvorrichtung für Lebensmittel
- 11: Gehäuse der Röntgen-Detektionsvorrichtung
- 12: Röntgen-Detektionsvorrichtung mit Röntgenquelle und Röntgendetektor
- 13: Produkte - Lebensmittel
- 14: Fördermittel - Förderband
- 15: Röntgenquelle
- 16: Röntgendetektor mit zeilenweiser Abtastung
- 17: von der Röntgenquelle abgegebene Röntgenstrahlung
- 18: Motor zum Antrieb des Förderbands
- 20: zentrale Steuerung für Röntgendetektor, Förderband und Positionserkennungsvorrichtung und Produktweiche
- 22: Produktweiche
- 24: Auswerteeinheit für den Röntgendetektor in der zentralen Steuerung
- 25: Positionserfassungseinrichtung
- 26: Display der zentralen Steuerung zur Anzeige von Fehlern und der Fehlerposition am Förderband
- 28: Verschmutzungen am Förderband, insb. aus Metall, z.B. Späne
- 30: Abtastzeile des Röntgendetektors - Erfassungsbereich des Röntgendetektors
- 32: Pixel einer Abtastzeile
- 34: Pixel mit einem um mehr als einen Grenzwert abweichenden Signalwert, der ein Warnsignal auslöst
- a: freier Bereich des Förderbandes zwischen den Produkten

## Patentansprüche

1. Vorrichtung (10) zur Erfassung von Fremdstoffen in Produkten (13), insbesondere Lebensmitteln, umfassend eine Röntgen-Detektionsvorrichtung (12) mit einer Röntgenquelle (15) und einem Röntgendetektor (16), und ein Fördermittel (14) zum Fördern der Produkte durch einen Erfassungsbereich (30) des Röntgendetektors (16),
wobei die Röntgen-Detektionsvorrichtung (12) ausgebildet ist, das Fördermittel (14) selbst ohne aufliegende Produkte (13) zu detektieren um ein pixelbasiertes Detektionsbild entsprechend der Auflösung des Röntgendetektors (16) zu erhalten,
wobei die Vorrichtung (10) eine Auswerteeinheit (24) aufweist, die ausgebildet ist, beim Kalibrieren der Vorrichtung (10) die Signalwerte jedes Pixels (32) des Röntgendetektors (16) auf einen Sollwert zu normieren,
wobei die Vorrichtung (10) weiterhin ausgebildet ist, im laufenden Betrieb mittels der Röntgen-Detektionsvorrichtung (12) produktfreie Bereiche des Fördermittels (14) zu scannen und Abweichungen der Signalwerte der in diesen Bereichen detektierten Pixel (32) von dem Sollwert zu erfassen und mittels der Auswertevorrichtung (24) auszuwerten, und ein Warnsignal abzugeben, wenn die Abweichung des Signalwerts eines oder mehrerer Pixel (34) in den produktfreien Bereichen (a) des Fördermittels (14) von dem Sollwert einen Grenzwert überschreitet.

2. Vorrichtung (10) nach Anspruch 1, **dadurch gekennzeichnet, dass** das Fördermittel (14) ein Förderband ist, auf welches die Produkte aufgelegt werden.

3. Vorrichtung (10) nach Anspruch 1 oder 2, **dadurch gekennzeichnet, dass** der Grenzwert sich auf die Anzahl der Pixel (34) mit vom Sollwert abweichenden Signalwerten bezieht.

4. Vorrichtung (10) nach einem der vorhergehenden Ansprüche, **dadurch gekennzeichnet, dass** die Auswerteeinheit (24) ausgebildet ist, eine Produktweiche (22) für die Produkte (13) anzusteuern.

5. Vorrichtung (10) nach einem der vorhergehenden Ansprüche, **dadurch gekennzeichnet, dass** der Röntgendetektors (16) ausgebildet ist, das Fördermittel (14) Zeile für Zeile zu erfassen, wobei jede Zeile in Pixel unterteilt ist.

6. Vorrichtung (10) nach einem der vorhergehenden Ansprüche, **dadurch gekennzeichnet, dass** sie ausgebildet ist, beim Kalibrieren der Röntgen-Detektionsvorrichtung (12) die Signalwerte jedes Pixels (32) mit einem Sollwert zu vergleichen und zur Erzielung des Sollwertes mit einem Verstärkungsfaktor zu verstärken, der die Differenz zwischen dem Signalwert und dem Sollwert widerspiegelt, wobei die Auswerteeinheit (24) ausgebildet ist, ein Warnsignal abzugeben, wenn der Signalwert des Pixels (32) nach der Normierung von dem Sollwert abweicht.

7. Vorrichtung (10) nach einem der vorhergehenden Ansprüche, **dadurch gekennzeichnet, dass** sie eine Positionserkennungseinrichtung (25) für die Position des Pixels (32) mit dem abweichenden Signalwert am Fördermittel (14) aufweist.

8. Vorrichtung (10) nach Anspruch 7, **dadurch gekennzeichnet, dass** Positionserkennungseinrichtung (25) ausgebildet ist, die aktuelle Position jedes Pixels des Röntgendetektors (16) in einer Abtastzeile und/oder die absolute Position des Fördermittels (14) zu erkennen.

9. Verfahren zur Detektion von Fremdstoffen in Produkten (13), insbesondere Lebensmitteln, die mittels eines Fördermittels (14) durch einen Erfassungsbereich (30) des Röntgendetektors (16) einer Röntgen-Detektionsvorrichtung (12) gefördert werden, **dadurch gekennzeichnet, dass** produktfreie Bereiche (a) des Fördermittels (14) von der Röntgen-Detektionsvorrichtung (12) im laufenden Betrieb detektiert und die Signalwerte pixelbasiert mit einem kalibrierten Sollwert verglichen werden, wobei ein Warnsignal abgegeben wird, wenn die Abweichung des Signalwertes zumindest eines Pixels (34) von dem kalibrierten Sollwert einen Grenzwert überschreitet.

10. Verfahren nach Anspruch 9, **dadurch gekennzeichnet, dass** der Grenzwert abhängig ist von der Anzahl der vom Sollwert abweichenden Pixel, insbesondere wenn die abweichenden Pixel nebeneinander liegen.

11. Verfahren nach Anspruch 9 oder 10, **dadurch gekennzeichnet, dass** das Fördermittel durch den Röntgendetektor (16) zeilenweise erfasst wird, wobei jede Zeile (30) wiederum in Pixel unterteil ist.

12. Verfahren nach einem der Ansprüche 9 bis 11, **dadurch gekennzeichnet, dass** beim Kalibrieren der Röntgen-Detektionsvorrichtung (12) die Signalwerte jedes Pixels (32) des Röntgendetektors (16) mit einem Sollwert verglichen und zur Erzielung des Sollwertes mit einem Verstärkungsfaktor verstärkt werden, der die Differenz zwischen dem Signalwert und dem Sollwert widerspiegelt, wobei ein Warnsignal abgegeben wird, wenn der Signalwert eines normierten Pixels (32) vom Sollwert abweicht.

13. Verfahren nach einem der Ansprüche 9 bis 12, **dadurch gekennzeichnet, dass** die des aktuelle Position eines Pixels des Röntgendetektors (16) in einer Abtastzeile (30) und/oder aktuelle Position des Fördermittels (14) ermittelt wird, und dass die Position eines fehlerhaften Bereichs (28) am Fördermittel (14), der das Warnsignal ausgelöst hat, angezeigt wird.

14. Verfahren nach einem der Ansprüche 9 bis 13, **gekennzeichnet durch** die Verwendung einer Vorrichtung (10) nach einem der Ansprüche 1 bis 8.
